# EUROPÄISCHE PATENTSCHRIFT

(11) **EP 0 601 223 B1**
(45) Veröffentlichungstag und Bekanntmachung des Hinweises auf die Patenterteilung: **12.11.1997**
(21) Anmeldenummer: 92120835.1
(22) Anmeldetag: 07.12.1992
(51) Int. Cl.: A61F 2/36

(54) **Schaft für eine femurale Hüftgelenk-Endoprothese**
Shaft for femoral hip joint endoprosthesis
Tige pour endoprothèse fémorale de d'articulation de la hanche

(43) Veröffentlichungstag der Anmeldung: 15.06.1994
(73) Patentinhaber: PLUS ENDOPROTHETIK AG, 6343 Rotkreuz (CH)
(72) Erfinder: Zweymüller,K., Prof. Dr.med., A-1180 Wien (AT); Deckner, André, F-75015 Paris (FR)
(74) Vertreter: Popp, Eugen, Dr.

(56) Entgegenhaltungen:
- EP-A- 0 207 873
- WO-A-90/13271
- US-A- 4 865 608
- US-A- 4 919 679

## Beschreibung

Die Erfindung betrifft einen Schaft für eine femurale Hüftgelenk-Endprothese gemäß Oberbegriff des Anspruches 1.

Ein derartiger Schaft für eine femurale Hüftgelenk-Endprothese ist in der EP-A-0 244 610 beschrieben. Dieser Schaft umfaßt ein im Querschnitt etwa rechteckförmiges Schaftblatt mit einem distalen und proximalen Ende. Dabei ist das distale Ende im wesentlichen konisch zulaufend ausgebildet. Das proximale Ende weist eine Einschlageinrichtung sowie eine Auszieheinrichtung zum Einschlagen des Schaftes in bzw. zum Herausziehen des Schaftes aus dem Markraum des Femurs auf. Des weiterem umfaßt dieser Schaft einen mit dem proximalem Ende des Schaftblattes über einen Hals verbunden Konus zur Aufnahme einer Gelenkkugel.

Als nachteilig bei diesem Schaft für eine femurale Hüftgelenk-Endprothese hat sich in der Praxis dessen Ausgestaltung der Einschlageinrichtung bzw. Auszieheinrichtung erwiesen. Als Einschlageinrichtung und Ausziehrichtung nämlich ist eine Bohrung in dem proximalem Ende vorgesehen, die einerseits senkrecht zur Mittelllängsachse des Schaftes und andererseits parallel zur Mittellängsebene des Schaftes verläuft. Zum Einschlagen des Schaftes in bzw. zum Herausziehen des Schaftes aus dem Markraum des Femurs ist ein im wesentlichen hackenförmig ausgebildetes Einschlag- und/oder Ausziehinstrument seitlich in die Bohrung von dem an dem proximalem Ende auf dem Hals angebrachten Konus her einführbar. Obschon durch eine solche konstruktive Ausgestaltung zusätzliche Knochenverluste im Trochanterbereich vermieden werden sollen, werden beim Einschlagen des Schaftes in den Markraum des Femurs aufgrund einer Krafteinleitung seitlich zur Mittellängsachse des Schaftblattes Kippmomente erzeugt, die zu einem lockeren Sitz des Schaftes im Markraum führen können. Darüber hinaus werden beim Herausziehen des Schaftes aus dem Markraum des Femurs ebenfalls infolge einer Kraftübertragung seitlich zur Mittellängsachse des Schaftblattes Kippmomente hervorgerufen, die das Entfernen des Implantates wesentlich erschweren. Schließlich war sowohl beim Einschlagen als auch beim Herausziehen des Schaftes in den bzw. aus dem Markraum des Femurs wegen der besonderen baulichen Ausgestaltung der Einschlageinrichtung und der Auszieheinrichtung häufig eine Beschädigung des speziellen Einschlag- bzw. Ausziehinstrumentes durch Bruch zu beobachten.

Abhilfe bezüglich der beschriebenen Nachteile der Einschlag- und Auszieheinrichtung gemäß EP-A-0 244 610 schafft eine Konstruktion, wie sie in der US-A-4,919,679 beschrieben und dargestellt ist. Dort weist die Einschlageinrichtung eine sich etwa quer zur Mittellängsachse des Schaftes erstreckende Aufsetzfläche für ein Einschlaginstrument auf. Die Aufsetzfläche ist etwa konkav und der sich ergebende Sattelpunkt fällt mit der Mittellängsachse zusammen. Auf diese Weise lassen sich Kippmomente beim Einschlagen und Herausziehen des Schaftes in den bzw. aus dem Femur vermeiden. Problematisch ist beim letztgenannten Stand der Technik jedoch die Handhabung; denn die Aufsetzfläche ist relativ klein innerhalb einer Bohrung geschaffen; dementsprechend muß der Opterateur das Instrumentarium regelrecht in diese Bohrung einfädeln. Darüber hinaus ist die Aufsetzfläche relativ klein mit der Gefahr einer Beschädigung aufgrund hoher Flächenpressung.

Der vorliegenden Erfindung liegt die Aufgabe zugrunde, einen Schaft für eine femurale Hüftgelenk-Endprothese der gattungsgemäßen Art bereit zu stellen, der unter Beibehaltung des Vorteils der Konstruktion gemäß der US-A-4,919,679 bezüglich einer koaxialen Krafteinleitung beim Einschlagen bzw. Herausziehen des Schaftes sich erheblich einfacher handhaben läßt.

Diese Aufgabe wird erfindungsgemäß durch die kennzeichnenden Merkmale des Anspruches 1 gelöst. Die erfindungsgemäße Konstruktion erleichtert das Ansetzen eines Einschlag- bzw. Ausziehinstruments ganz erheblich im Vergleich zum Stand der Technik nach der US-A-4,919,679 sowie EP-A-0 244 610. Die Aufsetzfläche ist relativ groß; dennoch wird eine Zentrierung des Einschlaginstruments erreicht, und zwar durch die Form der Aufsetzfläche selbst. Dadurch, daß die Schnittlinie der beiden Teilflächen der V-förmigen Aufsetzfläche mit der die Mittellängsachse enthaltenden Mittellängsebene des Schaftes zusammenfällt, wird eine exakt koaxiale Krafteinleitung beim Einschlagen des Schaftes erhalten. Dies führt zu einem qualitativ hohen Primärsitz des Schaftes und infolgedessen der Hüftgelenk-Endprothese insgesamt.

Die Maßnahme nach Anspruch 2, wonach die Einschlageinrichtung eine koaxial zur Mittellängsachse verlaufende Bohrung aufweist, die beispielsweise einen an dem Einschlaginstrument vorgesehenen Vorsprung, Stift oder dergleichen aufnimmt, wird eine zusätzliche Zentrierung erhalten. Vor allem wird dadurch gewährleistet, daß beim Einschlagvorgang das Einschlaginstrument nicht seitlich von der Aufsetzfläche wegrutscht.

Von Interesse sind des weiteren die Maßnahmen nach Anspruch 3, wonach die Auszieheinrichtung eine Bohrung mit einem Gewinde für ein Ausziehinstrument aufweist, deren Mittellängsachse mit der Mittellängsachse des Schaftes zusammenfällt. Durch die Bohrung mit dem Gewinde, die zum Beispiel mit einem als Ausziehschraube ausgebildeten Ausziehinstrument zusammenwirken kann, lassen sich ausgesprochen hohe, auf den im Markraum des Femurs eingesetzten Schaft einwirkende Ausziehkräfte erhalten und zwar ohne jegliche Gefahr einer Beschädigung des Ausziehinstrumentes selbst. Durch die besondere Anordnung der Bohrung gegenüber der Mittellängsachse des Schaftes sind zudem Kippmomente beim Herausziehen des Schaftes aus dem Markraum des Femurs ausgeschlossen.

Zur baulichen Vereinfachung sind die Bohrung zur Aufnahme des an einem Einschlaginstrument vorgesehenen Vorsprunges, Stiftes oder gleichen der Einschlageinrichtung und die Bohrung mit dem Gewinde für das Ausziehinstrument der Auszieheinrichtung gemäß Anspruch 4 identisch. Dabei ist der erste Gewindegang des Gewindes allerdings geringfügig in die Bohrung hineinverlegt, um das Gewinde selbst beim Einschlagvorgang nicht zu beschädigen.

Vorzugsweise besteht der Schaft aus Metall, insbesondere Titan oder einer Titanlegierung, beispielsweise aus der hochwertigen, geschmiedeten Titanlegierung Ti₆Al₇Nd.

Die mittlere Oberflächenrauhigkeit des Schaftes beträgt gemaß Anspruch 6 vorteilhafterweise 3 bis 5 µ. Diese Oberflächenrauhigkeit hat große Bedeutung für den Biomechanismus der Osteointegration des Femurs mit dem Schaft.

Nachstehend wird ein erfindungsgemäß ausbildeter Schaft anhand der beigefügten Zeichnung näher erläutert. Es zeigen:
- Fig. 1: eine perspektivische Seitenansicht einer Ausführungsform eines erfindungsgemäß ausgebildeten Schaftes;
- Fig. 2: eine Seitenansicht des Schaftes gemäß Fig. 1;
- Fig. 3: eine Rückansicht des Schaftes gemäß Fig. 1;
- Fig. 4: eine Vorderansicht des Schaftes gemäß Fig. 1;
- Fig. 5: eine Unteransicht des Schaftes gemäß Fig. 1;
- Fig. 6: eine Oberansicht des Schaftes gemäß Fig. 1; und
- Fig. 7: eine teilweise abgebrochene, perspektivische Seitenansicht einer anderen Ausführungsform eines erfindungsgemäß ausgebildeten Schaftes in vergrößertem Maßstab.

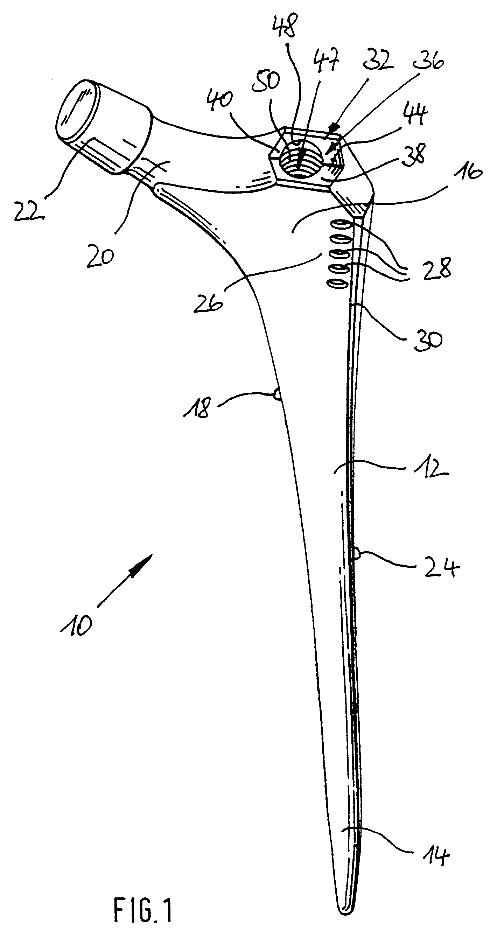

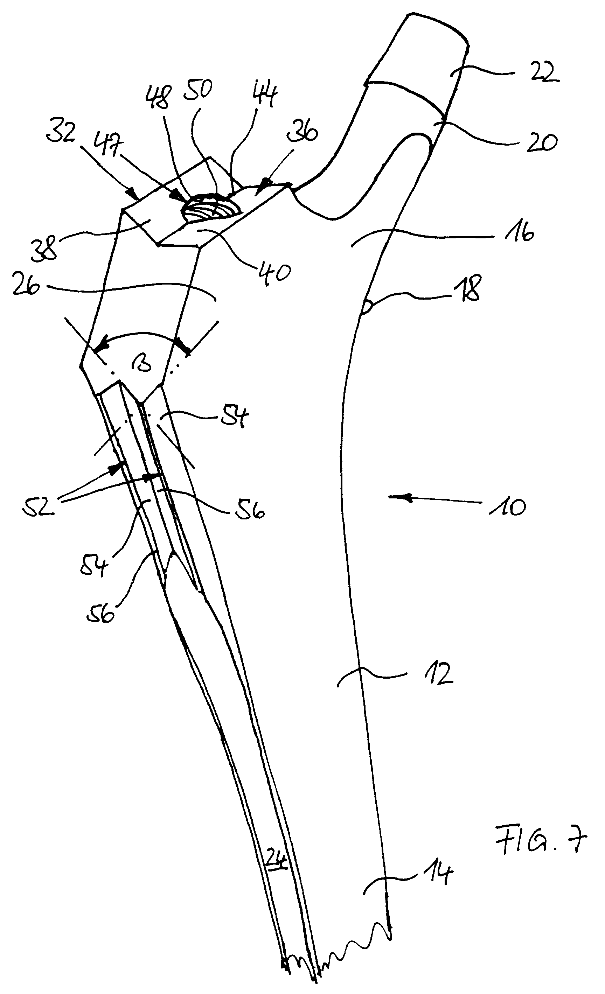

Die in den Fig. 1 bis 6 dargestellte Ausführungsform eines Schaftes 10 für eine femurale Hüftgelenk-Endoprothese umfaßt ein im Querschnitt etwa rechteckförmiges Schaftblatt 12. Die rechteckige Grundform des Schaftblattes 12 ermöglicht einerseits eine gute Rotationssicherung des Schaftes 10 in Verbindung mit einer großflächigen Kortikalisauflage, die eine ausgesprochen zuverlässige Primärstabilität des Schaftes 10 in dem Markraum eines nicht dargestellten Femurs hervorruft. Die rechteckige Grundform des Schaftblattes 12 aber besitzt andererseits den weiteren biologischen Vorteil, daß der Markraum vor Einsetzen des Schaftes nicht völlig ausgeräumt werden muß, wodurch eine ausreichende, nutritive Versorgung des Femurs aus dem Markraum als eine Vorraussetzung zur sekundären Osteointegration des Implantates erreicht wird.

Gleichzeitig ist das Schaftblatt 12, das ein distales Ende 14 und ein proximales Ende 16 aufweist, im wesentlichen konisch ausgestaltet. Demnach ist das distale Ende 14 des Schaftblattes 12 weitgehend konisch zulaufend ausgebildet. Das distale Ende 14 des Schaftblattes 12 ist dabei etwas abgerundet und geht kontinuierlich in eine Art Pyramidenspitze über. Auf diese Weise können punktuelle Spannungsspitzen am Femur vermieden werden, wodurch der Heilungsprozeß wie auch die Lebenserwartung der femuralen Hüftgelenk-Endoprothese insgesamt verbessert werden können.

Die mediale Schmalseite 18 des Schaftblattes 12 hat den Verlauf einer polynomischen Funktion, die dem Femur im Bereich des Calcarbogens nachgebildet ist. Auf diese Weise kann ein bei Wegnahme von Spongiosaknochen an größeren Partien des Calcarsmittels Raspelarbeit zwangsläufig entstehender Spalt beim nachfolgenden Einschlagen des Schaftes 10 ausgefüllt werden. Damit einhergehend wird die proximale Krafteinleitung wesentlich verbessert.

Die mediale Schmalseite 18 des Schaftblattes 12 geht im Bereich des proximalen Endes 16 über in einen Hals 20. Der Hals 20, der im Übergangsbereich zu dem proximalen Ende 16 des Schaftblattes 12 zur Vermeidung einer Bruchgefahr verstärkt ist, verjüngt sich hin zu dem mit dem Hals 20 verbundenen, zur Aufnahme einer nicht dargestellten Gelenkkugel vorgesehenen Konus 22. Durch eine solche Verjüngung des Halses 20 wird eine größere Beweglichkeit im künstlichen Hüftgelenk von bis zu 3° erreicht.

Die laterale Schmalseite 24 des Schaftblattes 12, die geringfügig konkav ausgebildet ist, geht im Bereich des proximalen Endes 16 des Schaftblattes 12 über in einen Trochanterflügel 26. Der Trochanterflügel 26 wirkt durch Vergrößerung der proximalen Verankerungsoberfläche im Femur besonders stabilisierend. Darüber hinaus ist der Trochanterflügel 26 bei der Knochenkompression in der Schlußphase des Einbringens des proximalen Endes 16 des Schaftblattes 12 von großer Bedeutung. So verhindert beispielsweise der Trochanterflügel 26 während des operativen Eingriffes oftmals in problematischen Fällen ein sog. Einsinken des Schaftblattes 12.

Im Trochanterflügel 26 selbst sind eine Reihe von Durchgangsbohrungen 28 oder dergleichen vorgesehen, die einerseits einer Beobachtung des Knochenmaterials bzw. der Knochenstruktur im Röntgenbild wie auch einer Identifikation des Schaftes 10 und andererseits einer zusätzlichen Verankerung durch mit der Zeit einwachsendes Knochenmaterial des Femurs dienen. Schließlich weist der Trochanterflügel 26 auf der lateralen Schmalseite 24 seitliche Abschrägungen 30 oder dergleichen auf.

Das proximale Ende 16 des Schaftblattes 12 von dem in den Fig. 1 bis 6 gezeigten Schaft 16 weist darüber hinaus eine Einschlageinrichtung 32 zum Einschlagen des Schaftes 10 in den Markraum des Femurs auf. Die Einschlageinrichtung 32 ist dabei an dem proximalen Ende 16 des Schaftblattes 12 im wesentlichen auf der Mittellängsachse 34 des Schaftes 10 für eine koaxiale Kraftübertragung angeordnet. Die Einschlageinrichtung 32 weist eine sich etwa quer zur Mittellängsachse 34 des Schaftes 10 erstreckende Aufsetzfläche 36 für ein nicht dargestelltes Einschlaginstrument auf.

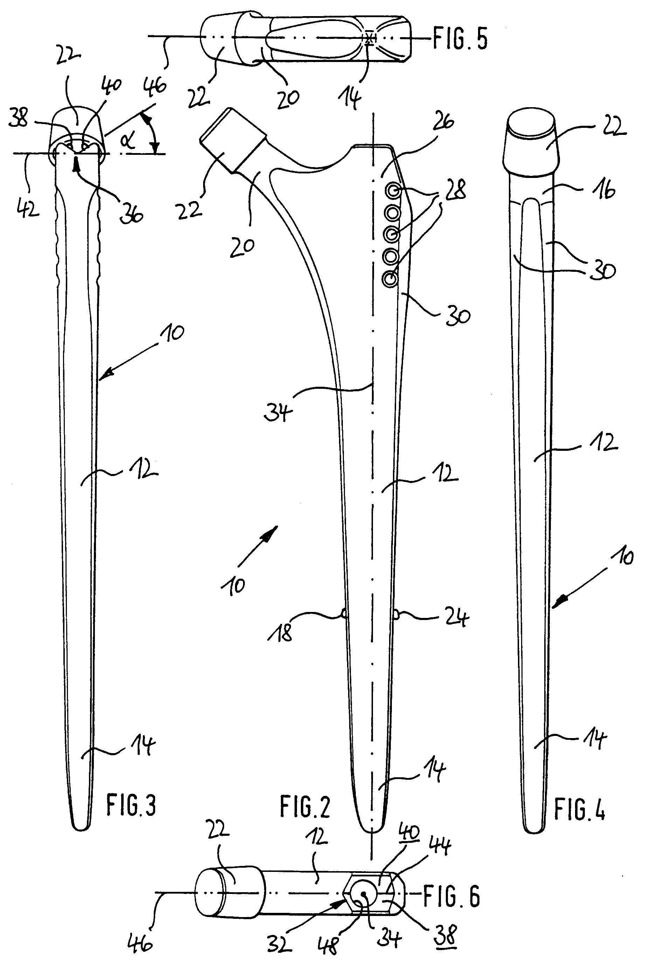

Die Aufsetzfläche 36 ist dabei V-förmig, dem distalen Ende 14 des Schaftblattes 12 zugewandt ausgebildet. Insbesondere ist die etwa V-förmige Aufsetzfläche 36 durch zwei Teilflächen 38, 40 zusammengesetzt, die jeweils gegenüber der sich senkrecht zur Blattebene erstreckenden Horizontalebene 42 um einen Winkel α von 15° bis 45°, vorzugsweise von 30° entsprechend Fig. 3, geneigt sind. Die Schnittlinie 44 der beiden Teilflächen 38, 40 der etwa V-förmigen Aufsetzfläche 36 fällt mit der sich ebenfalls senkrecht zur Blattebene erstreckenden Mittellängsebene 46 (vgl. Fig. 5 und 6) des Schaftes 10 zusammen.

Alternativ hierzu kann die Aufsetzfläche 36 auch etwa konkav, dem distalen Ende 14 des Schaftblattes 12 zugewandt ausgebildet sein, was in den Figuren allerdings nicht im einzelnen veranschaulicht ist. Die Sattellinie der etwa konkaven Aufsetzfläche 36 würde dann entsprechend der Schnittlinie 44 mit der Mittellängsebene 46 des Schaftes 10 zusammenfallen.

Die Einschlageinrichtung 32 kann des weiteren mit einer koaxial zur Mittellängsachse 34 verlaufenden Bohrung zur Aufnahme eines an dem Einschlaginstrument angebrachten Vorsprunges, Stiftes oder dergleichen versehen sein, um das Einschlaginstrument beim Einschlagen auf den Schaft 10 zusätzlich zu zentrieren und/oder zu fixieren und/oder festzulegen.

Das proximale Ende 16 des Schaftblattes 12 umfaßt weiterhin eine Auszieheinrichtung 47 zum Herausziehen des Schaftes 10 aus dem Markraum des Femurs. Die Auszieheinrichtung 47 ist ebenso wie die Einschlageinrichtung 32 an dem proximalen Ende 16 des Schaftblattes 12 im wesentlichen auf der Mittellängsachse 34 des Schaftes 10 für eine koaxiale Kraftübertragung angeordnet. Die Auszieheinrichtung 47 ist von einer Bohrung 48 mit einem Gewinde 50 für ein nicht dargestelltes Ausziehinstrument gebildet, wobei die Mittellängsachse der Bohrung 48 und die Mittellängachse 34 des Schaftes 10 zusammenfallen.

Bei dem Ausführungsbeispiel des Schaftes 10 nach den Fig. 1 bis 6 ist die Bohrung zur Aufnahme des an dem Einschlaginstrument vorgesehenen Vorsprunges, Stiftes oder dergleichen der Einschlageinrichtung und die Bohrung 48 mit dem Gewinde 50 für das Ausziehinstrument der Auszieheinrichtung 47 identisch. Um allerdings Beschädigungen des Gewindes 50 beim Einschlagen des Schaftes 10 in den Markraum des Femurs zu vermeiden, ist der erste Gewindegang des Gewindes 50 geringfügig in die Bohrung 48 hineinverlegt.

Eine weitere Ausführungsform eines Schaftes 10 ist in Fig. 7 dargestellt, die weitgehend mit derjenigen nach den Fig. 1 bis 6 übereinstimmt. Insofern sind gleiche Teile mit gleichen Bezugsziffern versehen.

Die Ausführungsform des Schaftes 10 nach Fig. 7 unterscheidet sich von derjenigen nach den Fig. 1 bis 6 lediglich durch zwei Längsrippen 52 oder dergleichen, die an der lateralen Schmalseite 24 nahe des Trochanterflügels 26 am proximalen Ende 16 des Schaftblattes 12 angeordnet sind. Die beiden Längsrippen 52, die etwa parallel zur Mittellängsachse 34 des Schaftes 10 verlaufen, dienen der zusätzlichen Führung des Schaftes 10 beim Einschlagen in den Markraum des Femurs bzw. beim Herausziehen aus diesem. Die Längsrippen 52 sind jeweils durch zwei etwa V-förmig zueinander angeordnete Teilflächen 54, 56 gebildet, die einen Winkel β von ca. 90° bis 120° einschließen.

Zur Verbesserung des Biomechanismus der Osteointegration des Femurs mit dem Implantat besteht der Schaft 10 aus Metall, insbesondere aus Titan oder einer Titanlegierung, wobei die Oberfläche des Schaftblattes 12 vorzugsweise eine mittlere Oberflächenrauhigkeit von 3 bis 5 µm besitzt.

## Patentansprüche

1. Schaft für eine femurale Hüftgelenk-Endoprothese, umfassend ein im Querschnitt etwa rechteckförmiges Schaftblatt (12) mit einem distalen und einem proximalen Ende (14, 16), wobei das distale Ende (14) im wesentlichen konisch zulaufend ausgebildet ist und wobei das proximale Ende (16) eine Einschlageinrichtung (32) sowie eine Auszieheinrichtung (47) zum Einschlagen des Schaftes (10) in den bzw. zum Herausziehen des Schaftes (10) aus dem Markraum des Femurs aufweist, und einen mit dem proxi-malen Ende (16) des Schaftblattes (12) über einen Hals (20) verbundenen Konus (22) zur Aufnahme einer Gelenkkugel,
**dadurch gekennzeichnet,**
daß die Einschlageinrichtung (32) und die Auszicheinrichtung (47) im wesentlichen auf der Mittellängsachse (34) des Schaftes (10) für eine koaxiale Kraftübertragung angeordnet sind, und
daß die Einschlageinrichtung (32) eine sich etwa quer zur Mittellängsachse (34) des Schaftes (10) erstreckende aus zwei ebenen Teilflächen (38,40) zusammengesetzte etwa V-förmige Aufsetzfläche (36) für ein Einschlaginstrument aufweist, deren Spitze dem distalen Ende des Schaftes (10) zugewandt ist, wobei die beiden Teilflächen (38, 40) der etwa V-förmigen Aufsetzfläche (36) gegenüber der senkrecht zur Schaft-Mittellängsachse (34) verlaufenden Horizontalebene (42) jeweils um einen Winkel α von 15° bis 45°, insbesondere von 30°, geneigt sind, und wobei, die Schnittlinie (44) der beiden Teilflächen (38, 40) mit der die Mittellängsachse (34) enthaltenden Mittellängsebene (46) des Schaftes (10) zusammenfällt.

2. Schaft nach Anspruch 1
**dadurch gekennzeichnet,**
daß die Einschlageinrichtung (32) eine koaxial zur Mittellängsachse (34) des Schaftes (10) verlaufende Bohrung zur Aufnahme eines an dem Einschlaginstrument zu dessen Zentrierung und/oder Fixierung vorgesehenen Vorsprunges, Stiftes oder dergleichen aufweist.

3. Schaft nach Anspruch 1 oder 2
**dadruch gekennzeichnet,**
daß die Aufzieheinrichtung (47) eine Bohrung (48) mit einem Gewinde (50) für ein Ausziehinstrument aufweist, wobei die Mittellängsachse der Bohrung (48) und die Mittellängsachse (34) des Schaftes (10) zusammenfallen.

4. Schaft nach Anspruch 2 und 3,
**dadurch gekennzeichnet**,
daß die Bohrung zur Aufnahme des an dem Einschlaginstrument vorgesehenen Vorsprunges, Stiftes oder dergleichen der Einschlageinrichtung und die Bohrung (48) mit dem Gewinde (50) für das Ausziehinstrument der Auszieheinrichtung (47) indentisch sind, wobei der erste Gewindegang des Gewindes (50) geringfügig in die Bohrung (48) hineinverlegt ist.

5. Schaft nach wenigstens einem der Ansprüche 1 bis 4 ,
**dadurch gekennzeichnet,**
daß der Schaft (10) aus Metall, insbesondere aus Titan oder einer Titanlegierung, besteht.

6. Schaft nach wenigstens einem der Ansprüche 1 bis 5
**dadurch gekennzeichnet,**
daß das Schaftblatt (12) eine mittlere Oberflächenrauhigkeit von 3 bis 5 µm aufweist.

## Claims

1. Stem for a femoral hip-joint endoprosthesis, comprising a stem blade (12) which is approximately rectangular in cross-section and which has a distal end and a proximal end (14, 16), the distal end (14) being essentially of a conically tapering design, and the proximal end (16) having an impaction arrangement (32) and an extraction arrangement (47) for driving the stem (10) into, and, respectively, removing the stem (10) from, the medullary cavity of the femur, and a cone (22) which is connected to the proximal end (16) of the stem blade (12) via a neck (20) and is used for receiving a joint ball, characterized in that the impaction arrangement (32) and the extraction arrangement (47) are arranged essentially on the central longitudinal axis (34) of the stem (10) for a coaxial force transmission, and in that the impaction arrangement (32) has an approximately V-shaped application surface (36) for an impaction instrument, which application surface (36) runs approximately transverse to the central longitudinal axis (34) of the stem (10) and is made up of two plane constituent surfaces (38, 40) and has its point directed towards the distal end of the stem (10), the two constituent surfaces (38, 40) of the approximately V-shaped application surface (36) each being inclined by an angle α of 15° to 45°, in particular of 30°, relative to the horizontal plane (42) running perpendicular to the central longitudinal axis (34) of the stem, and the line of intersection (44) of the two constituent surfaces (38, 40) coinciding with the central longitudinal plane (46) of the stem (10) which includes the central longitudinal axis (34).

2. Stem according to Claim 1, characterized in that the impaction arrangement (32) has a bore which runs coaxial to the central longitudinal axis (34) of the stem (10) and which is used for receiving a projection, pin or the like provided on the impaction instrument for the purpose of centring and/or fixing the latter.

3. Stem according to Claim 1 or 2, characterized in that the extraction arrangement (47) has a bore (48) with a thread (50) for an extraction instrument, the central longitudinal axis of the bore (48) and the central longitudinal axis (34) of the stem (10) coinciding.

4. Stem according to Claim 2 and 3, characterized in that the bore in the impaction arrangement for receiving the projection, pin or the like provided on the impaction instrument, and the bore (48) with the thread (50) in the extraction arrangement (47) for the extraction instrument are identical, with the first turn of the thread (50) being set slightly inwards into the bore (48).

5. Stem according to at least one of Claims 1 to 4, characterized in that the stem (10) is made of metal, in particular titanium or a titanium alloy.

6. Stem according to at least one of Claims 1 to 5, characterized in that the stem blade (12) has a mean surface roughness of 3 to 5 µm.

## Revendications

1. Tige pour endoprothèse fémorale de l'articulation de la hanche comprenant un fût de tige (12) de section transversale sensiblement rectangulaire avec une extrémité distale (14) et proximale (16), dans lesquelles l'extrémité distale (14) s'étend de manière sensiblement conique et l'extrémité proximale (16) présente un dispositif d'insertion (32) ainsi qu'un dispositif d'extraction (47) pour insérer, respectectivement extraire, la tige (10) dans ou hors de l'espace de moelle du fémur, ainsi qu'un cône (22) de réception de la boule d'articulation relié par un col (20) à l'extrémité proximale (16) du fût de tige (12)
caractérisée en ce que
les dispositifs d'insertion (32) et d'extraction (47) sont agencés sensiblement sur l'axe longitudinal médian (34) de la tige (10) pour une transmission de forces coaxiale, et en ce que
le dispositif d'insertion (32) présente, pour un instrument d'insertion, une surface d'assise (36) s'étendant sensiblement perpendiculairement à l'axe longitudinal médian (34) de la tige (10) et formée de deux parties de surfaces planes (38,40) sensiblement en forme de V dont la pointe est orientée vers l'extrémité distale de la tige (10), dans lesquelles les deux parties de surface (38,40) de la surface d'assise (36) sensiblement en forme de V sont inclinées respectivement d'un angle α de 15 à 45 degrés, en particulier de 30 degrés, par rapport à un plan horizontal (42) s'étendant perpendiculairement à l'axe longitudinal médian (34) de la tige, et dans lesquelles la ligne de séparation (44) des deux parties de surface (38,40) coïncident avec le plan longitudinal médian (46) contenant l'axe longitudinal médian (34) de la tige (10).

2. Tige selon la revendication 1, caractérisée en ce que le dispositif d'insertion (32) présente un perçage s'étendant coaxialement à l'axe longitudinal médian (34) de la tige (10) pour réception d'une protubérance, tenon ou analogue prévue sur l'instrument d'insertion pour centrage et/ou fixation.

3. Tige selon la revendication 1 ou 2, caractérisée en ce que le dispositif d'extraction (47) présente un perçage (48) avec un taraudage (50) pour l'instrument d'extraction, perçage dont l'axe longitudinal médian coïncide avec l'axe longitudinal médian (34) de la tige (10).

4. Tige selon la revendication 2 ou 3, caractérisée en ce que le perçage du dispositif d'insertion pour réception de la protubérance, tenon ou analogue prévue sur l'instrument d'insertion est identique au perçage (48) avec le taraudage (50) du dispositif d'extraction (47) pour l'instrument d'extraction, dans lequel la première entrée de filet du taraudage (50) est très peu décalé dans le perçage (48).

5. Tige selon l'une des revendication 1 à 4, caractérisée en ce que la tige (10) est réalisée en métal, en particulier en titane ou en alliage de titane.

6. Tige selon l'une des revendications 1 à 5, caractérisée en ce que le fût de tige (12) présente une rugosité surfacique moyenne de 3 à 5 µm.
